# FASCICULE DE BREVET EUROPEEN

(11) **EP 0 716 089 B1**
(45) Date de publication et mention de la délivrance du brevet: **20.08.1997**
(21) Numéro de dépôt: 95402275.2
(22) Date de dépôt: 11.10.1995
(51) Int. Cl.: C07F 7/08, C07F 7/21, C08G 77/388, A61K 7/42

(54) **Nouveaux filtres solaires, compositions cosmétiques photoprotectrices les contenant et utilisations**
Neue Sonnenschutzfilter, diese enthaltende kosmetische Sonnenschutzmittel und deren Verwendungen
Sunscreens, cosmetic sunscreen compositions and uses thereof

(30) Priorité: 17.11.1994 FR 9413769
(43) Date de publication de la demande: 12.06.1996
(73) Titulaire: L'OREAL, 75008 Paris (FR)
(72) Inventeur: Richard, Hervé, F-93420 Villepinte (FR); Leduc, Madeleine, F-75011 Paris (FR); Lagrange, Alain, F-77700 Couvray (FR)
(74) Mandataire: Andral, Christophe André Louis

(56) Documents cités:
- WO-A-92/19625
- JOURNAL OF ORGANOMETALLIC CHEMISTRY, vol. 285, 1985 pages 375-381, FITZMAURICE, N.J. ET AL. 'THE STEREOCHEMISTRY OF ORGANOMETALLIC COMPOUNDS. XXXVI. REGIO- AND STEREO-CHEMICAL CONTROL IN THE NICKEL-CATALYSED HYDROCYANATION OF SILYLALKYNES'

## Description

La présente invention concerne de nouveaux composés du type diorganosiloxanes, à chaines courtes, linéaires ou cycliques, ou du type triorganosilanes, présentant pour caractéristique commune d'avoir tous au moins un motif filtrant dérivé d'un cinnamonitrile à fonction alkylène ou alkylèneoxy, ces composés étant plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux composés destinés à la photoprotection (UV-A et/ou UV-B) de la peau ont été proposés à ce jour. Des composés du type organosiloxanes à fonction benzotriazole utilisables à titre de filtres solaires sont décrits dans le brevet WO92/19625.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber selectivement les rayonnements UV nocifs, ces filtres (et leurs quantités) étant sélectionnés en fonction de l'indice de protection recherché (l'indice de protection (lP) s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant intrinsèque, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence). Il est bien entendu également souhaitable que ces substances filtrantes ne pénètrent pas dans la peau, et soient non toxiques.

Parmi tous les composés aromatiques qui ont été préconisés à cet effet, nombre d'entre eux ne présentent pas toutes les propriétés requises pour une utilisation convenable comme filtres UV dans les compositions antisolaires. En particulier, leur pouvoir filtrant intrinsèque peut être insuffisant, et de ce fait, afin d'obtenir des propriétés filtrantes satisfaisantes, il est souvent nécessaire de mettre en oeuvre des quantités relativement importantes de produits, ce qui se traduit par de mauvaises propriétés cosmétiques au niveau des formulations finales qui les contiennent ; en outre, leur solubilité dans les différents type de formulations utilisés en matière de protection solaire n'est pas toujours suffisamment bonne (liposolubilité notamment) ; ils peuvent par ailleurs ne pas posséder une stabilité suffisante à la lumière (photostabilité) ; et ils peuvent enfin également présenter une mauvaise résistance à l'eau et à la sueur.

La présente invention vise à proposer une nouvelle famille de composés, plus particulièrement utilisables à titre de filtres solaires pour compositions cosmétiques à effets anti-UV, et ne présentant pas les inconvénients ci-dessus.

Ainsi, selon la présente invention, il a été trouvé qu'en venant fixer par greffage, notamment selon une réaction d'hydrosylilation, un ou plusieurs motifs filtrants particuliers, à savoir des dérivés de cinnamonitriles, sur une chaine silicone convenablement sélectionnée, linéaire ou cyclique, ou sur un silane convenablement sélectionné, et ceci par. l'intermédiaire d'un chainon de raccordement spécifique de type alkylène ou alkylèneoxy, il était possible d'aboutir à de nouveaux composés à propriétés remarquables, ces nouveaux composés présentant en particulier de très bonnes propriétés filtrantes, soit dans l'UV-A soit dans l'UV-B selon leur structure chimique, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

Cette découverte est à la base de la présente invention.

La présente invention a ainsi pour premier objet de nouveaux composés qui sont caractérisés par le fait qu'ils répondent à l'une des formules (1) à (3) suivantes : 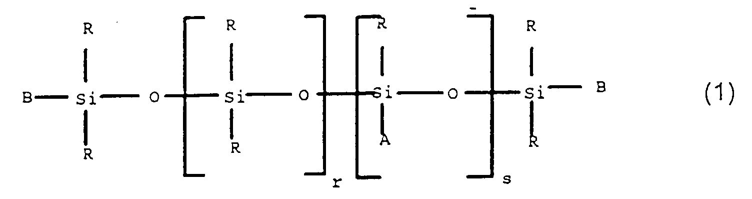 ou 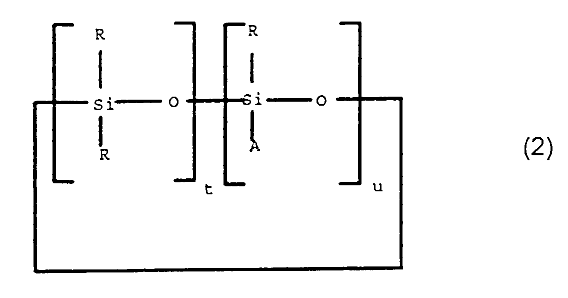 ou

A-Si(R')₃ (3)

formules (1) à (3) dans lesquelles :
- R, identiques ou différents, sont choisis parmi les radicaux alkyles en C₁-C_{10,} phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (4) suivante : 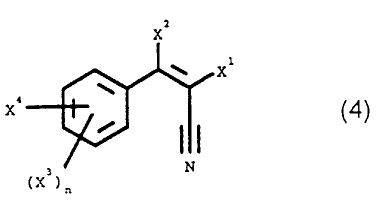
formule (4) dans laquelle :
* **X**^{**1**} représente un radical cyano ou un radical alcoxy carbonyle COOR¹ dans lequel le radical R¹ désigne (i) un radical alkyle en C₁-C₂₀ ou (ii) un radical hydroxyalkyle en C₂-C₂₀ ou encore (iii) un radical divalent -Y- de formule (5) suivante : 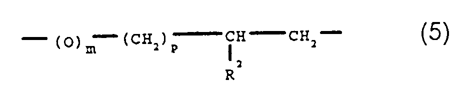 formule (5) dans laquelle :
   - R² désigne un atome d'hydrogène ou un radical alkyle en C₁-C₄,
   - p est un nombre entier compris entre 0 et 10 inclusivement,
   - m est 0 ou 1
   - le groupement -CH₂- terminal est directement lié à un atome de silicium ;
* **X**^{**2**} représente un atome d'hydrogène ou un radical phényle, éventuellement substitué, en C₆-C₁₀ ;
* **n** est un nombre entier compris inclusivement entre 0 et 2 ;
* **X**^{**3**}, identiques ou différents, sont choisis parmi les radicaux alkyles en C₁-C₈ ou les radicaux alcoxy en C₁-C₄, étant entendu que dans ce dernier cas deux X³ adjacents (n=2) peuvent former ensemble un groupement alkylidène dioxy dans lequel le groupe alkylidène contient 1 ou 2 atomes de carbone ;
* **X**^{**4**} représente un atome d'hydrogène ou un radical divalent -Y-,
étant entendu, dans cette formule (4), que si X⁴ représente un atome d'hydrogène alors X¹ doit nécessairement représenter un radical COOY dans lequel m est nul.

Dans les formules (1) à (3) ci-dessus, A représente donc le groupement dérivé du cinnamonitrile qui, après fixation sur la chaine courte siliconée de départ ou sur le silane de départ, confère aux composés de type diorganosiloxanes linéaires (formule (1)) ou cycliques (formule (2)) ou de type triorganosilanes (formule (3)) des propriétés absorbantes vis à vis de l'ultraviolet dans une zone de longueur d'onde pouvant aller de 280 à 400 nm. Comme indiqué précédemment, et comme cela ressort de la définition de la formule (4) donnée ci-dessus, ce groupement présente nécessairement au moins une fonction alkylène ou alkylèneoxy correspondant au chainon qui assure l'accrochage du cinnamonitrile à la chaine silicone ou au silane. L'un des avantages des composés selon l'invention est que l'on peut obtenir, selon la nature et/ou la position des différents groupements portés sur le motif filtrant A, des filtres soit purement UV-A ou au contraire purement UV-B, et ceci avec des coefficients d'extinction particulièrement élevés.

Comme cela ressort des définitions données ci-avant, l'accrochage du chainon -(O)ₘ-(CH₂)ₚ-CH(R²)-CH₂- (c'est à dire du radical divalent -Y-) sur le motif dérivé du cinnamonitrile, qui assure donc le raccordement dudit motif à l'atome de silicium de la chaine siliconée ou du silane, peut, selon la présente invention, se faire à l'une quelconque des positions occupées par les symboles X¹ ou X⁴, la partie terminale -(O)ₘ- dudit chainon venant se fixer sur le motif dérivé du cinnamonitrile et sa partie terminale -CH₂- sur un atome de silicium de la chaine siliconée ou du silane.

Dans les formules (1) à (3) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R, R' et B préférés selon l'invention sont les radicaux méthyle, éthyle, propyle, n-butyle, n-octyle et éthyl-2 hexyle. Encore plus préférentiellement, les radicaux R, R' et B sont tous des radicaux méthyle.

Parmi les composés de formules (1) à (3) ci-dessus, on préfère mettre en oeuvre ceux répondant à la formule (1) ou à la formule (2), c'est à dire des diorganosiloxanes à chaine courte, linéaire ou cyclique.

Parmi les diorganosiloxanes linéaires ou cycliques rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement méthyle,
- B est alkyle et encore plus préférentiellement méthyle (cas des composés linéaires de formule (1)),
- r est compris entre 0 et 6 inclusivement ; s est compris entre 0 et 6 inclusivement (cas des composés linéaires de formule (1)),
- t+u est compris entre 3 et 5 (cas des composés cycliques de formule (2)),
- X¹ est cyano, ou un radical COOR¹ dans lequel R¹ est méthyle, éthyle ou -Y-,
- X² est H ou phényle,
- X³ est méthoxy (n ≠ 0) ou H (n=0),
- X⁴ est -Y-, de préférence en position 4 du cycle
- p est compris inclusivement entre 1 et 3,
- R² est H ou méthyle.

Pour préparer les filtres siliconés de formule (1) et (2), on peut procéder classiquement (voie 1) en mettant en oeuvre une réaction d'hydrosylilation 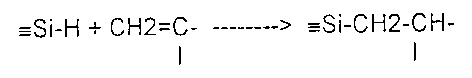 à partir de la silicone correspondante dans laquelle, par exemple, tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709.

Ce dérivé à SiH peut être donc représenté, selon les cas, soit par la formule (1bis) suivante : 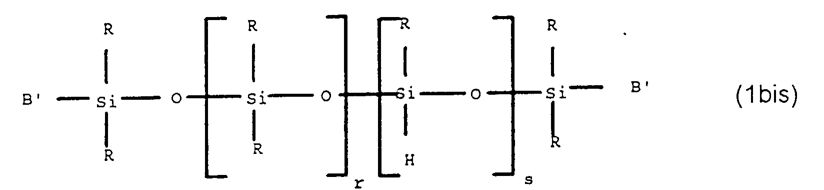 dans laquelle R, r et s ont la signification donnée ci-dessus pour la formule (1) et les radicaux B', identiques ou différents, sont choisis parmi les radicaux R et un atome d'hydrogène,
soit par la formule (2bis) suivante : 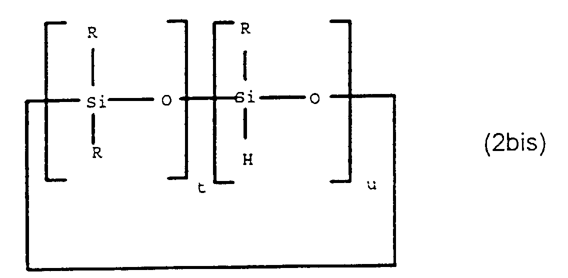 dans laquelle R, t et u ont la signification donnée ci-dessus pour la formule (2).

Sur ce dérivé à SiH de formules (1bis) ou (2bis), on effectue donc une réaction d'hydrosilylation classique, opérée en présence d'une quantité catalytiquement efficace d'un catalyseur au platine, sur un dérivé organique de cinnamonitrile choisi parmi ceux de formule (4bis) suivante : 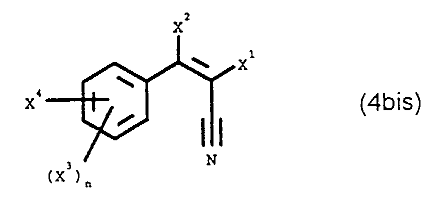 dans laquelle X¹, X², X³, X⁴ et n ont la même signification que celle donnée ci-avant pour la formule (4), à cette seule différence près que l'un des deux radicaux X¹ ou X⁴, au lieu de représenter un radical divalent -Y- de formule (5) définie ci-avant, représente ici le radical monovalent homologue insaturé correspondant, de formule (5bis) suivante : 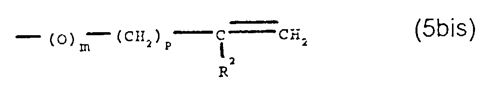 dans laquelle R², m et p ont la même signification qu'à la formule (5).

Parmi les composés de formule (4bis) ci-dessus rentrant particulièrement bien dans le cadre de la présente invention, on peut notamment citer les :
a) Cyano-2 diphényl-3,3 acrylate de méthyl-2 allyle
b) (Allyloxy-4 phényl)-3 cyano-2 acrylate d'éthyle
c) Cyano-2 [méthoxy-3 (méthyl-2 allyloxy)-4 phényl] acrylate d'éthyle
d) [Allyloxy-4 méthoxy-3 phényl]-3 cyano-2 acrylate d'éthyle.

Des procédés convenant à la préparation des produits de formule (4bis) ci-dessus sont notamment décrits dans les demandes de brevets FR-A- 1 487 593 et EP-A- 0 430 023 et dans le brevet US 4 178 303, dont les enseignements sont, à cet égard, totalement inclus dans la présente demande à titre de références. En particulier, ces produits peuvent être obtenus par réaction du para allyloxy benzaldéhyde avec le cyano acrylate d'éthyle (réaction dite de "Knoevenagel").

Les catalyseurs au platine utilisés pour réaliser la réaction d'hydrosylilation entre les composés de formule (1bis) ou (2bis) ci-dessus avec le composé de formule (4bis) ci-dessus sont bien connus et amplement décrits dans la littérature. On peut en particulier citer les complexes du platine et d'un produit organique décrit dans les brevets américains US-A- 3 159 601, US-A- 3 159 602, US-A- 3 220 972 et les demandes de brevets européens EP-A- 0 057 459, EP-A- 0 188978 et EP-A-0 190 530 et les complexes du platine et d'organopolysiloxane vinylé décrits dans les brevets américains US-A- 3 419 593, US-A- 3 377 432 et US-A- 3 814 730. Pour faire réagir les composés de formule (1bis) ou (2bis) avec le composé de formule (4bis), on utilise généralement une quantité de catalyseur au platine, calculée en poids de platine métal, comprise entre 5 et 600 ppm, de préférence entre 10 et 200 ppm, basée sur le poids de composés de formule (1bis) ou (2bis). La réaction d'hydrosylilation peut avoir lieu en masse ou au sein d'un solvant organique volatil tel que le toluène, l'heptane, le xylène, le tétrahydrofuranne et le tétrachloréthylène. Il est généralement souhaitable de chauffer le mélange réactionnel à une température comprise entre 60 et 120°C pendant le temps pour que la réaction soit complète. On peut ajouter goutte à goutte le composé de formule (1bis) ou (2bis) sur le composé de formule (4bis) en solution dans un solvant organique contenant le catalyseur. On peut aussi ajouter simultanément le composé de formule (1bis) ou (2bis) et le composé de formule (4bis) à une suspension de catalyseur dans un solvant organique. Il est recommandé de vérifier que la réaction est complète en dosant les SiH résiduels par la potasse alcoolique, puis on élimine le solvant, par exemple par distillation sous pression réduite. L'huile brute obtenue peut être purifiée, par exemple par passage sur une colonne absorbante de silice.

Concernant la préparation des filtres de type triorganosilanes de formule (3) donnée précédemment, on peut procéder comme indiqué ci-dessus, toujours par réaction d'hydrosylilation, entre un silane de départ de formule (R')₃Si-H (formule (3bis), dans laquelle R' a la même signification que pour le composé de formule (3), et un dérivé organique de cinnamonitrile de formule (4bis) définie ci-avant.

Une autre voie de synthèse possible (voie 2) convenant à la préparation des filtres siliconés de formules (1) et (2) dans le cas particulier où X² représente l'hydrogène, consiste à conduire une réaction d'hydrosylilation entre un composé de formule respectivement (1bis) ou (2bis) définies ci-avant avec cette fois un dérivé formylé insaturé choisi parmi ceux de formule (4ter) suivante : 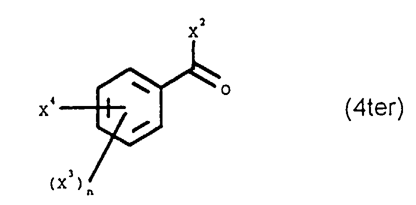 dans laquelle X² est Hydrogène, n et X³ ont la même signification que celle donnée ci-avant pour la formule (4), et X⁴ représente un radical monovalent insaturé de formule (5bis) définie ci-avant.

Le produit obtenu après hydrosylilation est ensuite mis en réaction avec un cyano acétate ou le malonitrile de formule (6) suivante : 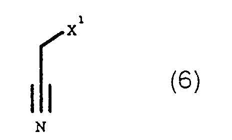 dans laquelle X¹ désigne un radical cyano ou un radical alkoxy carbonyle COOR¹ dans lequel R¹ peut désigner un radical alkyle linéaire ou ramifié en C₁-C₂₀ ou un radical hydroxyalkyle en C₂-C₂₀.

ce par quoi l'on obtient finalement le composé de formule (1) ou (2) désiré.

Comme indiqué précédemment, les composés de formules (1) à (3) ci-dessus présentent un excellent pouvoir filtrant intrinsèque à l'égard du rayonnement ultraviolet (UV-A ou UV-B, selon la structure du produit). En mélangeant des produits de structure différente, c'est à dire plus précisément en mélangeant des produits conformes à l'invention présentant une activité purement UV-A avec des produits conformes à l'invention présentant une activité purement UV-B, il est ainsi possible de disposer d'une composition qui présentera globalement une activité filtrante dans toute la gamme des UV nocifs (UV-A + UV-B), ce qui constitue un avantage important. En outre, de par leur caractère fortement liposoluble, les composés de formule (1) à (3) ci-dessus peuvent être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. Enfin, leurs propriétés cosmétiques sont très bonnes, à savoir en particulier que ces produits, par rapport aux silicones filtres de l'art antérieur, sont moins collants et apportent plus de douceur.

La présente invention a donc aussi pour objet une composition cosmétique comprenant, dans un support cosmétiquement acceptable contenant de préférence au moins une phase grasse ou un solvant organique, une quantité efficace d'au moins un composé de formules (1) à (3) définies ci-avant.

Les composés de formules (1) à (3) sont généralement présents dans des proportions comprises entre 0,1 % et 20 % en poids, de préférence entre 0,5 % et 10 % en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Elle peut contenir les adjuvants cosmétiques habituellement utilisés dans le domaine, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des sequestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.
Les corps gras peuvent être constitués par une huile ou par une cire ou leurs mélanges, des acides gras, des esters d'acides gras, des alcools gras, la vaseline, la paraffine, la lanoline, la lanolinehydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères. Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet les composés de formule (1), (2) ou (3) tels que définis ci-avant ou les compositions définies ci-dessus pour leur utilisation dans un procédé de protection de la peau et/ou des cheveux contre le rayonnement ultraviolet, en particulier le rayonnement solaire, qui consiste à appliquer sur la peau ou les cheveux une quantité efficace d'au moins un de ces composés ou une de ces compositions.

Les exemples qui suivent illustrent l'invention sans toutefois en limiter la portée.

### EXEMPLE 1

Cet exemple illuste la préparation (selon la voie 1) d'un composé conforme à l'invention répondant à la formule : 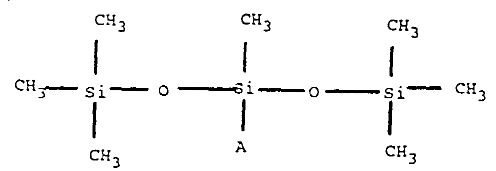 dans laquelle A représente : 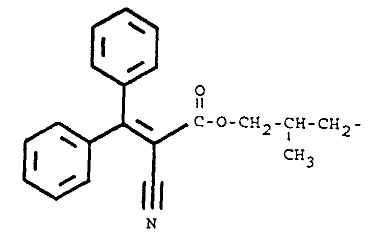 (ce produit correspond à un composé de formule (1) pour lequel R = B = CH₃ ; r = 0 ; s = 1 ; n = 0 ; X² = phényle ; X⁴ = H ; X¹ = COO-Y- avec pour Y : m=0, p=1 et R²= CH₃).

### a) Première étape :

Dans un réacteur de 250 ml, on introduit de la benzophénone (50 g ; 0,274 mole), du cyano acetate de méthallyle (46 g ; 0,33 mole), de l'acétate d'ammonium (4,8 g), de l'acide acétique (14,4 g) et de l'heptane (110 ml). On chauffe le mélange au reflux et on élimine l'eau par distillation. Toutes les heures, on rajoute 1,5 g d'un mélange d'acide acétique/acétate d'ammonium 75:25 . On maintient l'agitation et le chauffage pendant un temps total de 24 heures. On refroidit et verse le mélange dans l'eau, puis l'extrait au dichlorométhane. Après lavage à l'eau et évaporation du solvant, on purifie sur colonne sous pression (éluant : heptane/acétate d'éthyle 95:5). On récupère dans les premières fractions la benzophénone de départ (24 g), puis on obtient dans les autres fractions 28,5 g de Cyano-2 diphényl-3,3 acrylate de méthyl-2 allyle, de caractéristiques suivantes :
Huile jaune pâle

| Analyse élémentaire : | | | |
|---|---|---|---|
| théorie | C79,19 | H5,65 | N4,62 |
| trouvé | C79,57 | H5,59 | N4,51 |

### b) Deuxième étape :

Dans un réacteur, on charge 14,5 g du produit obtenu précédemment et 30 ml de toluène. Le mélange est ensuite porté à 80°C sous azote. On ajoute le catalyseur d'hydrosylilation (complexe à 3-3,5% de Pt dans du cyclovinylméthylsiloxane de Hüls Petrarch PC085 : 30 µl) puis, goutte à goutte et en 20 minutes, 11,7 g d'heptaméthyltrisiloxane. Après 3 heures à 80°C sous azote, on concentre le milieu réactionnel et on effectue une chromatographie sur Silice sous pression (éluant : heptane/EtOAC 97:3). On obtient alors 9,5 g du produit final désiré, de caractéristiques suivantes :
huile jaune pâle

| Analyse élémentaire : | | | | |
|---|---|---|---|---|
| calculé | C61,67 | H7,48 | N2,66 | Si16,02 |
| trouvé | C61,79 | H7,59 | N2,51 | Si15,89 |

Les caractéristiques en absorption UV (mesurée dans l'éthanol) de ce produit sont les suivantes :
λₘₐₓ : 303 nm εₘₐₓ : 11 950 Ce produit peut donc être utilisé très efficacement à titre de filtre solaire actif dans l'UV- B.

### EXEMPLE 2

Cet exemple illuste la préparation (selon la voie 2) d'un composé conforme à l'invention répondant à la formule : 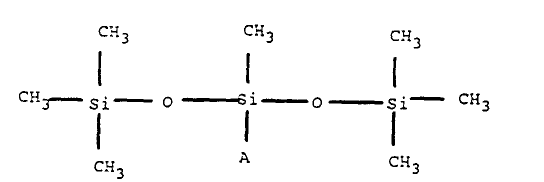 dans laquelle A représente : 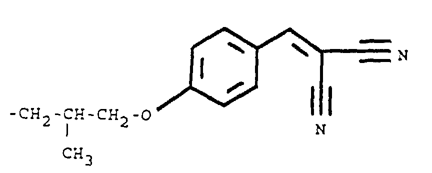 (ce produit correspond à un composé de formule (1) pour lequel R = B = CH₃ ; r = 0 ; s = 1 ; X¹ = cyano ; X² = H ; n = 0 ; X⁴ = -Y- avec pour Y : p=m=1 et R²= méthyle).

### a) Première étape :

Dans un réacteur, on charge 17,62 g (0,1 mole) de (méthyl-2 allyloxy)-4 benzaldéhyde et 50 ml de toluène sec. Le mélange est porté à 80°C sous azote. On ajoute ensuite le catalyseur d'hydrosylilation (complexe à 3-3,5% de Pt dans du cyclovinylméthylsiloxane de Hüls Petrarch PC085 : 100 µl) puis, goutte à goutte et en 20 minutes, 24,47 g (0,11 mole) d'heptaméthyltrisiloxane. Après 6 heures à 80°C sous azote, on concentre le milieu réactionnel et on effectue une chromatographie sur Silice sous pression (éluant : heptane/EtOAc 98:2). On obtient alors 34,1 g (Rendement:85%) de [tétraméthyl-1,3,3,3 (triméthylsilyl)oxy-1 disiloxanyl]-3 méthyl-2 propyloxy benzaldéhyde, de caractéristiques suivantes :
Huile jaune pâle
Température d'ébullition = 135-141°C sous 0.1 mmHg

### b) Deuxième étape :

Dans un réacteur, on place 3,7 g du produit obtenu précédemment, 2 ml d'éthanol, 0,62 g de malonitrile et 2 gouttes de pipéridine. On laisse sous agitation à température ordinaire pendant 4 heures. On concentre sous vide et le produit obtenu est recristallisé dans le méthanol pour donner le produit final désiré, de caractéristiques suivantes:
Poudre blanche à reflets jaunes
Pf: 41-42°C

| Analyse élémentaire : | | | | |
|---|---|---|---|---|
| calculé | C56,46 | H7,67 | N6,27 | Si18,86 |
| trouvé | C56,44 | H7,65 | N5,94 | Si18,55 |

Les caractéristiques en absorption UV (mesurée dans l'éthanol) de ce produit sont les suivantes :
λₘₐₓ : 351 nm εₘₐₓ : 32 400 Ce produit peut donc être utilisé très efficacement à titre de filtre solaire actif dans l'UV- A.

### EXEMPLE 3

En suivant le même mode opératoire que celui donné soit à l'exemple 1 soit à l'exemple 2, on a préparé quatre autres composés conformes à l'invention (composés A à C selon le procédé de l'exemple 1 ; composé D selon le procédé de l'exemple 2), rentrant tous dans le cadre de la formule générale (1) et se différenciant les uns des autres par leur squelette siliconé et/ou la nature du motif filtrant A.

Les formules de ces composés A à D étaient ainsi les suivantes ;

### Composé A :

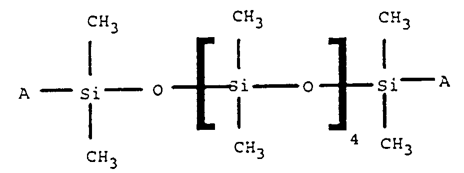

avec pour A : 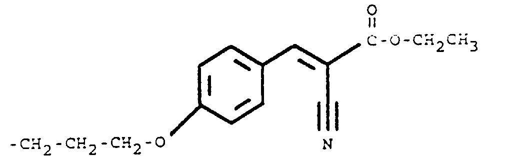 (ce produit correspond à un composé de formule (1) pour lequel R = CH₃ ; B = A; r = 4 ; s = 0 ; n = 0 ; X¹ = COOR¹ avec R¹ = éthyle ; X² = H ; X⁴ = -Y- avec pour Y : m = p = 1 et R² = H).

### Composé B :

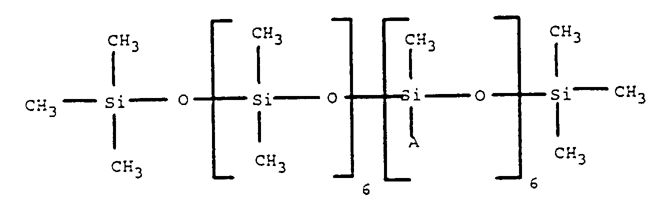

avec pour A : 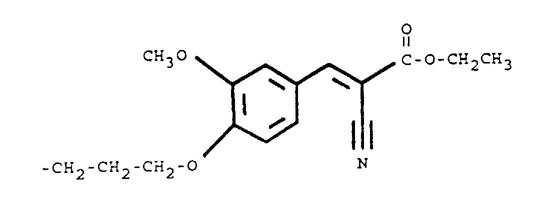 (ce produit correspond à un composé de formule (1) pour lequel R = B = CH₃ ; r (valeur moyenne) = s (valeur moyenne) = 6 ; X¹ = COOR¹ avec R¹ = éthyle X² = H ; n = 1 et X³ = méthoxy ; X⁴ = -Y- avec pour Y : m = p = 1 et R² = H).

### Composé C :

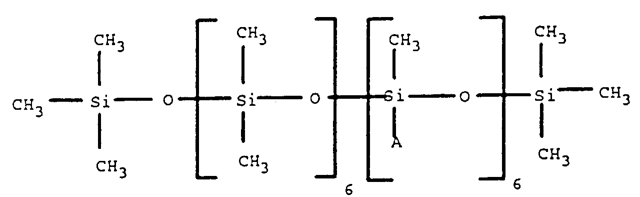

avec pour A : 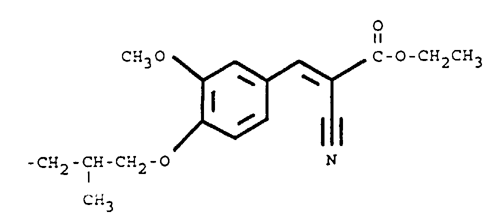 (ce produit correspond à un composé de formule (1) pour lequel R = B = CH₃ ; r (valeur moyenne) = s (valeur moyenne) = 6 ; X¹ = COOR¹ avec R¹ = éthyle ; X² = H ; n = 1 et X³ = méthoxy ; X⁴ = -Y- avec pour Y : m = p = 1 et R² = CH₃).

### Composé D :

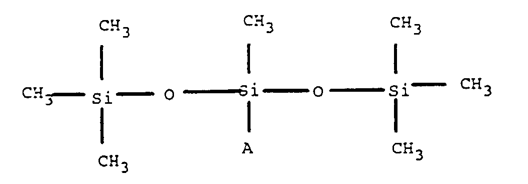

avec pour A : 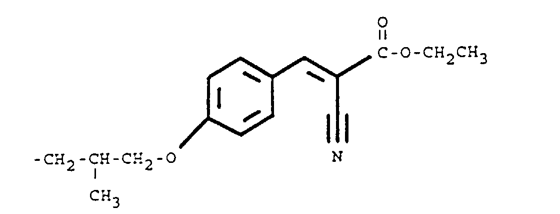 (ce produit correspond à un composé de formule (1) pour lequel R = B = CH₃ ; r = 0 ; s = 1 ; X¹ = COOR¹ avec R¹ = éthyle ; X² = H ; n = 0 ;
X⁴ = -Y- avec pour Y : m = p = 1 et R²= CH₃)

Pour chacun des composés A à D préparés, on a obtenu les résultats qui sont rassemblés dans le tableau ci-dessous.

**TABLEAU**

| Composé | **UV** (solvant) | | **Analyse Elémentaire** |
|---|---|---|---|
| | λₘₐₓ | εₘₐₓ | (1) = calculé (2) = trouvé |
| | 348nm | | (1): C53,35 H7,25 N2,96 Si17,82 |
| **A** | | E1%*=590 | |
| | (CHCl3) | | (2): C53,63 H7,09 N2,72 Si17,54 |
| | 364nm | | rmn du ¹H et du ²⁹Si en accord avec la formule |
| **B** | | E1%*=450 | |
| | (CHCl3) | | |
| | 365nm | | rmn du ¹H et du ²⁹Si en accord avec la formule |
| **C** | | E1%*=265 | |
| | (CHCl3) | | |
| | 344nm | | (1): C55,94 H7,96 N2,84 Si17,06 |
| **D** | | 31 000 | |
| | (éthanol) | | (2): C55,97 H7,92 N2,78 Si16,85 |

| | | | |
|---|---|---|---|
| * : E1 % désigne la densité optique d'une solution à 1 % de produit. | | | |

Les composés A, B, C et D peuvent donc être utilisés très efficacement à titre de filtre solaire actif dans l'UV- A.

### EXEMPLE 4

On illustre ici une formulation concrète de composition cosmétique antisolaire conforme à l'invention, à savoir une crème antisolaire.
- Composé de l'exemple 1 5 g
- Mélange d'alcool cétylstéarylique et d'alcool cétylstéarylique oxyéthyléné à 33 moles d'OE ("SINNOVAX AO" de chez HENKEL) 7 g
- Mélange de mono et distéarate de glycérol non autoémulsifiable 2 g
- Alcool cétylique 1,5 g
- Benzoate d'alcools en C₁₂-C₁₅ ("FINSOLV TN" de chez WITCO) 20 g
- Polydiméthylsiloxane 1,5 g
- Glycérine 17,5 g
- Parfum, conservateur qs
- Eau qsp 100 g
Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

## Revendications

1. Composés de formules (1) à (3) suivantes : 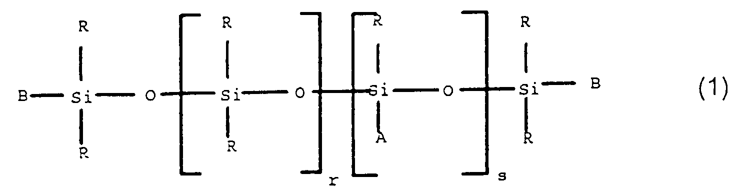 ou 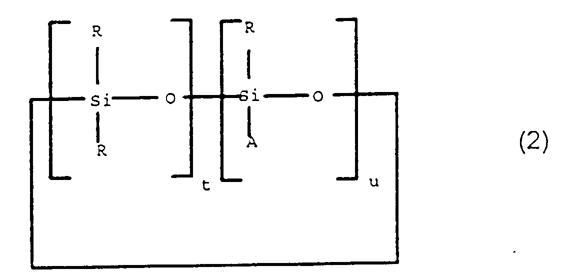 ou
A-Si(R')₃ (3)
formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (4) suivante : 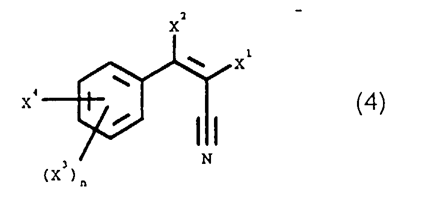
formule (4) dans laquelle :
* **X**^{**1**} représente un radical cyano ou un radical alcoxy carbonyle COOR¹ dans lequel le radical R¹ désigne (i) un radical alkyle en C₁-C₂₀ ou (ii) un radical hydroxyalkyle en C₂-C₂₀ ou encore (iii) un radical divalent -Y- de formule (5) suivante : 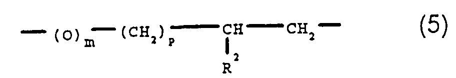 formule (5) dans laquelle :
- R² désigne un atome d'hydrogène ou un radical alkyle en C₁-C₄,
- p est un nombre entier compris entre 0 et 10 inclusivement,
- m est 0 ou 1
- le groupement -CH₂- terminal est directement lié à un atome de silicium ;
* **X**^{**2**} représente un atome d'hydrogène ou un radical phényle, éventuellement substitué, en C₆-C₁₀ ;
* **n** est un nombre entier compris inclusivement entre 0 et 2 ;
* **X**^{**3**}, identiques ou différents, sont choisis parmi les radicaux alkyles en C₁-C₈ ou les radicaux alcoxy en C₁-C₄, étant entendu que dans ce dernier cas deux X³ adjacents (n=2) peuvent former ensemble un groupement alkylidène dioxy dans lequel le groupe alkylidène contient 1 ou 2 atomes de carbone ;
* **X**^{**4**} représente un atome d'hydrogène ou un radical divalent -Y-,
étant entendu, dans cette formule (4), que si X⁴ représente un atome d'hydrogène alors X¹ doit nécessairement représenter un radical COOY dans lequel m est nul.

2. Composés selon la revendication 1, répondant à la formule (1) ou à la formule (2), caractérisés par le fait que les radicaux R sont des radicaux alkyle.

3. Composés selon la revendication 2, caractérisés par le fait que les radicaux R sont des radicaux méthyle, éthyle, propyle, n-butyle, n-octyle ou éthyl-2 hexyle.

4. Composés selon la revendication 3, caractérisés par le fait que les radicaux R sont des radicaux méthyle.

5. Composés selon l'une quelconque des revendications précédentes, répondant à la formule (1), caractérisés par le fait que les radicaux B sont des radicaux alkyle.

6. Composés selon la revendication 5, caractérisés par le fait que les radicaux B sont des radicaux méthyle, éthyle, propyle, n-butyle, n-octyle ou éthyl-2 hexyle.

7. Composés selon la revendication 6, caractérisés par le fait que les radicaux B sont radicaux méthyle.

8. Composés selon l'une quelconque des revendications précédentes, répondant à la formule (1), caractérisés par le fait que r est compris entre 0 et 6 inclusivement, et s est compris entre 0 et 6 inclusivement.

9. Composés selon l'une quelconque des revendications 1 à 4, répondant à la formule (2), caractérisés par le fait que t + u est compris entre 3 et 5 inclusivement.

10. Composés selon la revendication 1, répondant à la formule (3), caractérisés par le fait que les radicaux R' sont des radicaux alkyle choisis parmi les radicaux méthyle, éthyle, propyle, n-butyle, n-octyle ou éthyl-2 hexyle.

11. Composés selon la revendication 10, caractérisés par le fait que les radicaux R' sont des radicaux méthyle.

12. Composés selon l'une quelconque des revendications précédentes, caractérisés par le fait que X¹ est un radical cyano, ou un radical COOR¹ dans lequel R¹ est méthyle, éthyle ou -Y-.

13. Composés selon l'une quelconque des revendications précédentes, caractérisés par le fait que X² est l'hydrogène ou un radical phényle.

14. Composés selon l'une quelconque des revendications précédentes, caractérisés par le fait que X³ est un radical méthoxy (n≠0) ou l'hydrogène (n=0).

15. Composés selon l'une quelconque des revendications précédentes, caractérisés par le fait que X⁴ est un radical divalent -Y-.

16. Composés selon la revendication 15, caractérisés par le fait que ledit radical divalent -Y- est en position 4 du cycle benzénique.

17. Composés selon l'une quelconque des revendications précédentes, caractérisés par le fait que dans le radical divalent -Y-, p est compris inclusivement entre 1 et 3.

18. Composés selon l'une quelconque des revendications précédentes, caractérisés par le fait que dans le radical divalent -Y-, R² représente l'hydrogène ou un radical méthyle.

19. Composés selon l'une quelconque des revendications 1 à 18 pour leur utilisation comme filtres solaires actifs dans le domaine des UV-A et/ou UV-B.

20. Composition cosmétique, en particulier pour la filtration des rayons ultraviolets, caractérisée par le fait qu'elle comprend, dans un support cosmétiquement acceptable, une quantité efficace d'au moins un des composés définis à l'une quelconque des revendications 1 à 19.

21. Composition cosmétique selon la revendication 20, caractérisée par le fait que ledit support cosmétiquement acceptable contient au moins une phase grasse ou un solvant organique.

22. Composition cosmétique selon la revendication 21, caractérisée par le fait que ledit support se présente sous la forme d'une émulsion de type huile-dans-eau ou eau-dans-huile, de préférence huile-dans-eau.

23. Composition cosmétique selon l'une des revendications 20 à 23, caractérisée par le fait que la teneur en composé(s) filtrant(s) est comprise entre 0,1 et 20 % en poids par rapport au poids total de la composition.

24. Composition cosmétique selon la revendication 23, caractérisée par le fait que ladite teneur est comprise entre entre 0,5 et 10 % en poids.

25. Composés selon l'une quelconque des revendications 1 à 19 pour leur utilisation dans un procédé de protection de la peau et/ou des cheveux contre le rayonnement ultraviolet, en particulier le rayonnement solaire, consistant à appliquer sur la peau et/ou les cheveux une quantité efficace d'au moins un de ces composés.

26. Compositions selon l'une quelconque des revendications 20 à 24 pour leur utilisation dans un procédé de protection de la peau et/ou des cheveux contre le rayonnement ultraviolet, en particulier le rayonnement solaire, consistant à appliquer sur la peau et/ou les cheveux une quantité efficace d'au moins une de ces compositions.

## Patentansprüche

1. Neue Verbindungen der folgenden Formeln (1) bis (3): 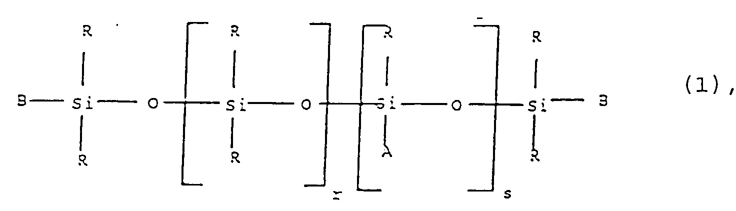 oder 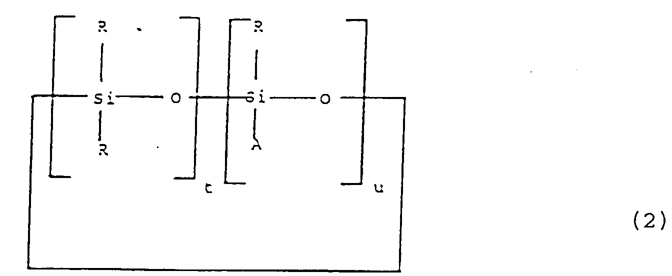 oder
A-Si(R')₃ (3),
wobei in den Formeln (1) bis (3)
- die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Gruppen R Methyl bedeuten,
- die Gruppen B, die identisch oder voneinander verschieden sind, unter den oben genannten Gruppen R und der nachfolgend definierten Gruppe A ausgewählt sind,
- die Gruppen R', die identisch oder voneinander verschieden sind, unter den C₁₋₈-Alkylgruppen und Phenyl ausgewählt sind,
- r eine ganze Zahl von Null bis einschließlich 50 und s eine ganze Zahl von Null bis einschließlich 20 bedeutet, mit der Maßgabe, daß mindestens eine der beiden Gruppen B A bedeutet, wenn s Null ist,
- u eine ganze Zahl im Bereich von 1 bis einschließlich 6 und t eine ganze Zahl im Bereich von Null bis einschließlich 10 bedeutet, mit der Maßgabe, daß u + t mindestens 3 ist,
- die Gruppe A eine einwertige Gruppe bedeutet, die direkt an ein Siliciumatom gebunden ist und der folgenden Formel (4) entspricht: 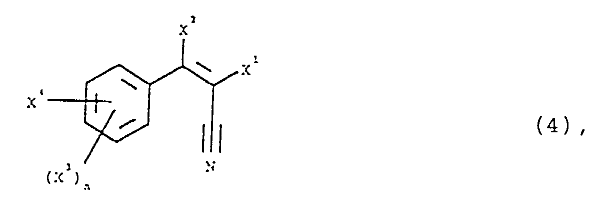 wobei in der Formel (4)
- X¹ eine Cyanogruppe oder Alkoxycarbonylgruppe COOR¹ bedeutet, wobei R¹ bedeutet:
(i) eine C₁₋₂₀-Alkylgruppe,
(ii) eine C₂₋₂₀-Hydroxyalkylgruppe oder
(iii) eine zweiwertige Gruppe -Y- der folgenden Formel (5) 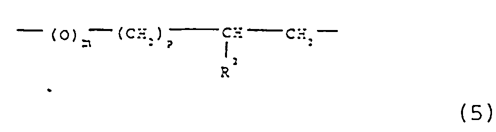 wobei in der Formel (5)
- R² Wasserstoff oder C₁₋₄-Alkyl bedeutet,
- p eine ganze Zahl im Bereich von Null bis einschließlich 10 ist,
- m Null oder 1 ist,
- die -CH₂-Endgruppe direkt an ein Siliciumatom gebunden ist,
- X² Wasserstoff oder eine gegebenenfalls C₆₋₁₀-substituierte Phenylgruppe bedeutet,
- n eine ganze Zahl im Bereich von Null bis einschließlich 2 ist,
- X³, die identisch oder voneinander verschieden sind, unter den C₁₋₈-Alkylgruppen oder C₁₋₄-Alkoxygruppen ausgewählt sind, mit der Maßgabe, daß im letzten Fall zwei aneinandergrenzende X³ (n=2) gemeinsam eine Alkylidendioxygruppe bilden können, wobei die Alkylidengruppe 1 oder 2 Kohlenstoffatome enthält,
- X⁴ Wasserstoff oder eine zweiwertige Gruppe -Y- bedeutet,
mit der Maßgabe, daß in der Formel (4) X¹ notwendigerweise eine Gruppe COOY mit m = Null bedeuten muß, wenn X⁴ Wasserstoff bedeutet.

2. Neue Verbindungen nach Anspruch 1, die der Formel (1) oder (2) entsprechen, dadurch gekennzeichnet, daß die Gruppen R Alkylgruppen bedeuten.

3. Neue Verbindungen nach Anspruch 2, dadurch gekennzeichnet, daß die Gruppen R Methyl, Ethyl, Propyl, n-Butyl, n-Octyl oder 2-Ethylhexyl bedeuten.

4. Neue Verbindungen nach Anspruch 3, dadurch gekennzeichnet, daß die Gruppen R Methyl bedeuten.

5. Neue Verbindungen nach einem der vorhergehenden Ansprüche, die der Formel (1) entsprechen, dadurch gekennzeichnet, daß die Gruppen B Alkyl bedeuten.

6. Neue Verbindungen nach Anspruch 5, dadurch gekennzeichnet, daß die Gruppen B Methyl, Ethyl, Propyl, n-Butyl, n-Octyl oder 2-Ethylhexyl bedeuten.

7. Neue Verbindungen nach Anspruch 6, dadurch gekennzeichnet, daß die Gruppen B Methyl bedeuten.

8. Neue Verbindungen nach einem der vorhergehenden Ansprüche, die der Formel (1) entsprechen, dadurch gekennzeichnet, daß r im Bereich von 1 bis einschließlich 6 und s im Bereich von 0 bis einschließlich 6 liegt.

9. Neue Verbindungen nach einem der Ansprüche 1 bis 4, dadurch gekennzeichnet, daß t + u im Bereich von 3 bis einschließlich 5 liegt.

10. Neue Verbindungen nach Anspruch 1, dadurch gekennzeichnet, daß die Gruppen R' Alkylgruppen sind, die unter Methyl, Ethyl, Propyl, n-Butyl, n-Octyl oder 2-Ethylhexyl ausgewählt sind.

11. Neue Verbindungen nach Anspruch 10, dadurch gekennzeichnet, daß die Gruppen R' Methyl bedeuten.

12. Neue Verbindungen nach einem der vorhergehenden Ansprüche, dadurch gekennzeichnet, daß X¹ Cyano oder eine Gruppe COOR¹ bedeutet, worin R¹ Methyl, Ethyl oder -Y- ist.

13. Neue Verbindungen nach einem der vorhergehenden Ansprüche, dadurch gekennzeichnet, daß X² Wasserstoff oder Phenyl bedeutet.

14. Neue Verbindungen nach einem der vorhergehenden Ansprüche, dadurch gekennzeichnet, daß X³ Methoxy (n ≠ 0) oder Wasserstoff (n = 0) bedeutet.

15. Neue Verbindungen nach einem der vorhergehenden Ansprüche, dadurch gekennzeichnet, daß X⁴ eine zweiwertige Gruppe -Y- bedeutet.

16. Neue Verbindungen nach Anspruch 15, dadurch gekennzeichnet, daß sich die zweiwertige Gruppe -Y- in 4-Stellung des Benzolrings befindet.

17. Neue Verbindungen nach einem der vorhergehenden Ansprüche, dadurch gekennzeichnet, daß in der zweiwertigen Gruppe -Y- p im Bereich von 1 bis einschließlich 3 liegt.

18. Neue Verbindungen nach einem der vorhergehenden Ansprüche, dadurch gekennzeichnet, daß in der zweiwertigen Gruppe -Y- R² Wasserstoff oder Methyl bedeutet.

19. Verbindungen nach einem der Ansprüche 1 bis 18 zur Verwendung als im UV-A-Bereich und/oder UV-B-Bereich wirksame Sonnenschutzfilter.

20. Kosmetische Zusammensetzungen, insbesondere um UV-Strahlung zu filtern, dadurch gekennzeichnet, daß sie in einem kosmetisch akzeptablen Träger eine wirksame Menge mindestens einer Verbindung nach einem der Ansprüche 1 bis 19 enthalten.

21. Kosmetische Zusammensetzungen nach Anspruch 20, dadurch gekennzeichnet, daß der kosmetisch akzeptable Träger mindestens eine Fettphase oder ein organisches Lösungsmittel enthält.

22. Kosmetische Zusammensetzungen nach Anspruch 21, dadurch gekennzeichnet, daß der Träger in Form einer Öl-in-Wasser- oder Wasser-in-Öl-Emulsion und vorzugsweise in Form einer Öl-in-Wasser-Emulsion vorliegt.

23. Kosmetische Zusammensetzungen nach einem der Ansprüche 20 bis 23, dadurch gekennzeichnet, daß der Mengenanteil der Filterverbindung(en) im Bereich von 0,1 bis 20 Gew.-%, bezogen auf das Gesamtgewicht der Zusammensetzung, liegt.

24. Kosmetische Zusammensetzungen nach Anspruch 23, dadurch gekennzeichnet, daß der Mengenanteil im Bereich von 0,5 bis 10 Gew.-% liegt.

25. Verbindungen nach einem der Ansprüche 1 bis 19 zur Verwendung in einem Verfahren zum Schutz der Haut und/oder der Haare gegen UV-Strahlung und insbesondere Sonnenlicht, das darin besteht, auf die Haut und/oder die Haare eine wirksame Menge mindestens einer dieser Verbindungen aufzutragen.

26. Zusammensetzungen nach einem der Ansprüche 20 bis 24 zur Verwendung in einem Verfahren zum Schutz der Haut und/oder der Haare gegen UV-Strahlung und insbesondere Sonnenlicht, das darin besteht, auf die Haut und/oder die Haare eine wirksame Menge mindestens einer dieser Verbindungen aufzutragen.

## Claims

1. Novel compounds of following formulae (1) to (3): 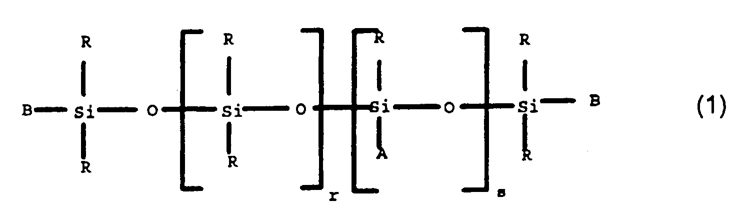 or 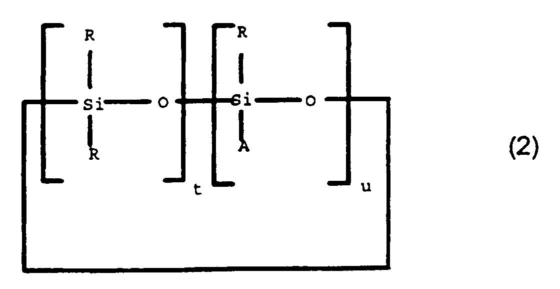 or
A-Si(R')₃ (3)
in which formulae (1) to (3):
- the groups R, which may be identical or different, are chosen from C₁-C₁₀ alkyl, phenyl and 3,3,3-trifluoropropyl radicals, at least 80 %, in numerical terms, of the radicals R being methyl,
- the groups B, which may be identical or different, are chosen from the above radicals R and the radical A defined below,
- the groups R', which may be identical or different, are chosen from C₁-C₈ alkyl radicals, or phenyl radicals,
- r is an integer between 0 and 50 inclusive, and s is an integer between 0 and 20 inclusive, with the condition that if s is zero then at least one of the two symbols B denotes A,
- u is an integer between 1 and 6 inclusive, and t is an integer between 0 and 10 inclusive, it being understood that t + u is equal to or greater than 3,
- and the symbol A denotes a monovalent radical directly attached to a silicon atom, and which corresponds to the following formula (4): 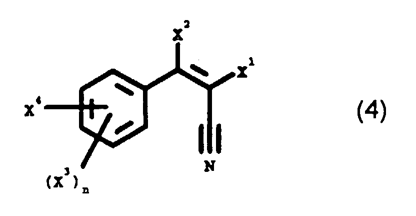
in which formula (4):
* **X**^{**1**} represents a cyano radical or an alkoxycarbonyl radical COOR¹ in which the radical R¹ denotes (i) a C₁-C₂₀ alkyl radical or (ii) a C₂-C₂₀ hydroxyalkyl radical or alternatively (iii) a divalent radical -Y- of following formula (5): 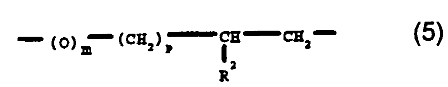 in which formula (5):
- R² denotes a hydrogen atom or a C₁-C₄ alkyl radical,
- p is an integer between 0 and 10 inclusive,
- m is 0 or 1,
- the end -CH₂- group is directly attached to a silicon atom;
* **X**^{**2**} represents a hydrogen atom or an optionally substituted C₆-C₁₀ phenyl radical;
* **n** is an integer between 0 and 2 inclusive;
* the groups **X**^{**3**}**,** which may be identical or different, are chosen from C₁-C₈ alkyl radicals and C₁-C₄ alkoxy radicals, it being understood that in the latter case two adjacent X³ groups (n=2) may together form an alkylidenedioxy group in which the alkylidene group contains 1 or 2 carbon atoms;
* **X**^{**4**} represents a hydrogen atom or a divalent radical -Y-,
it being understood that, in this formula (4), if X⁴ represents a hydrogen atom then X¹ must necessarily represent a radical COOY in which m is zero.

2. Novel compounds according to Claim 1, corresponding to formula (1) or to formula (2), characterized in that the radicals R are alkyl radicals.

3. Novel compounds according to Claim 2, characterized in that the radicals R are methyl, ethyl, propyl, n-butyl, n-octyl or 2-ethylhexyl radicals.

4. Novel compounds according to Claim 3, characterized in that the radicals R are methyl radicals.

5. Novel compounds according to any one of the preceding claims, corresponding to formula (1), characterized in that the radicals B are alkyl radicals.

6. Novel compounds according to Claim 5, characterized in that the radicals B are methyl, ethyl, propyl, n-butyl, n-octyl or 2-ethylhexyl radicals.

7. Novel compounds according to Claim 6, characterized in that the radicals B are methyl radicals.

8. Novel compounds according to any one of the preceding claims, corresponding to formula (1), characterized in that r is between 0 and 6 inclusive, and s is between 0 and 6 inclusive.

9. Novel compounds according to any one of Claims 1 to 4, corresponding to formula (2), characterized in that t + u is between 3 and 5 inclusive.

10. Novel compounds according to Claim 1, corresponding to formula (3), characterized in that the radicals R' are alkyl radicals chosen from the methyl, ethyl, propyl, n-butyl, n-octyl and 2-ethylhexyl radicals.

11. Novel compounds according to Claim 10, characterized in that the radicals R' are methyl radicals.

12. Novel compounds according to any one of the preceding claims, characterized in that X¹ is a cyano radical or a radical COOR¹ in which R¹ is methyl, ethyl or -Y-.

13. Novel compounds according to any one of the preceding claims, characterized in that X² is hydrogen or a phenyl radical.

14. Novel compounds according to any one of the preceding claims, characterized in that X³ is a methoxy radical (n≠0) or hydrogen (n=0).

15. Novel compounds according to any one of the preceding claims, characterized in that X⁴ is a divalent radical -Y-.

16. Novel compounds according to Claim 15, characterized in that the said divalent radical -Y- is in position 4 of the benzene ring.

17. Novel compounds according to any one of the preceding claims, characterized in that, in the divalent radical -Y-, p is between 1 and 3 inclusive.

18. Novel compounds according to any one of the preceding claims, characterized in that, in the divalent radical -Y-, R² represents hydrogen or a methyl radical.

19. Compounds according to any one of Claims 1 to 18 for their use as sunscreens which are active in the UV-A and/or UV-B region.

20. Cosmetic composition, in particular for screening out ultraviolet rays, characterized in that it comprises, in a cosmetically acceptable vehicle, an effective amount of at least one of the compounds defined in any one of Claims 1 to 19.

21. Cosmetic composition according to Claim 20, characterized in that the said cosmetically acceptable vehicle contains at least one fatty phase or one organic solvent.

22. Cosmetic composition according to Claim 21, characterized in that the said vehicle is in the form of an emulsion of oil-in-water or water-in-oil type, preferably of oil-in-water type.

23. Cosmetic composition according to one of Claims 20 to 23, characterized in that the content of screening compound(s) is between 0.1 and 20 % by weight relative to the total weight of the composition.

24. Cosmetic composition according to Claim 23, characterized in that the said content is between 0.5 and 10 % by weight.

25. Compounds according to any one of Claims 1 to 19 for their use in a process for the protection of the skin and/or the hair against ultraviolet radiation, in particular solar radiation, which process consists in applying to the skin and/or the hair an effective amount of at least one of these compounds.

26. Compositions according to any one of Claims 20 to 24 for their use in a process for the protection of the skin and/or the hair against ultraviolet radiation, in particular solar radiation, which process consist in applying to the skin and/or the hair an effective amount of at least one of these compositions.
